Europäisches Patentamt

European Patent Office

Office européen des brevets

(19)

(11) **EP 1 440 982 A2**

(12) **EUROPEAN PATENT APPLICATION**
published in accordance with Art. 158(3) EPC

(43) Date of publication:
28.07.2004 Bulletin 2004/31

(21) Application number: 02759834.1

(22) Date of filing: 20.03.2002

(51) Int Cl.⁷: **C07K 14/56**, C07K 14/575,
C12N 15/86, C07K 19/00,
C12N 15/62, A61K 38/21,
A61K 38/22, A61P 31/12,
A61P 37/04

(86) International application number:
**PCT/IB2002/002100**

(87) International publication number:
**WO 2002/081519 (17.10.2002 Gazette 2002/42)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **21.03.2001 CN 1105705X**

(71) Applicant: **Genemedix PLC
Suffolk CB8 8RX (GB)**

(72) Inventor: **WU, Xiangfu
Shanghai 200031 (CN)**

(74) Representative:
**Wright, Robert Gordon McRae et al
Elkington and Fife LLP,
Prospect House,
8 Pembroke Road
Sevenoaks, Kent TN13 1XR (GB)**

(54) **IFN-THY FUSION PROTEIN, DNA CODING THEREFORE, ITS PREPARATION AND APPLICATION**

(57) This invention provides a fusion protein of Interferon and Thymosin (IFN-THY fusion protein), a DNA sequence coding for this fusion protein, a vector containing this DNA sequence, a host cell containing this vector, a method for preparing this fusion protein using genetic engineering, and pharmaceutical compositions containing this fusion protein. The IFN-THY fusion protein of this invention has the activity of IFN and also has the immuno-excitation activity of THY, and it can be used for the treatment of such illnesses as viral infections and for strengthening the immune function of the organism.

Figure 1.

EP 1 440 982 A2

**Description**

This invention concerns recombinant DNA techniques and the area of pharmaceutics. In more concrete terms, this invention concerns a fusion protein of human α interferon 2b (HuIFN α 2b, hereinafter abbreviated to IFN) and human thymosin (hereinafter abbreviated to THY), a DNA sequence coding for this fusion protein, a vector containing this DNA sequence, a host cell containing this vector, a method for preparing this fusion protein using genetic engineering, and the use of this fusion protein for the treatment of such illnesses as viral infections and for strengthening the immune function of the organism.

Interferon was first discovered in 1957, when Isaaca and Lindemann quoted it as being capable of inducing the production of cell products antagonistic to homologous or heterogenic viral infections. After more than 40 years of research, the two main categories of interferon (IFN-α/β, also known as Type I IFN and IFN-γ also known as Type II IFN) genes, protein structure and its receptor genes, and protein structure have all received fairly deep study. During this period many biological activities of IFN were discovered, such as inducing an anti-viral state, inhibiting cell growth, stimulating cell growth, activation of natural killer cells etc. However, of these its anti-viral activity is the most generally known. IFN-α is produced by leukocytes and has a relatively strong action in inhibiting viral reproduction, in inhibiting tumour cell replication and in immunity adjustment. IFN-α has now been authorised for use in the treatment of many illnesses such as viral infections.

Capon D. J. et al reported that HuIFNα 2b can raise immune performance and so provide anti-viral therapeutic effects (Capon D. J., Shepard H. M. and Goeddel D. V., Mol. Cell. Biol. 5 (4), 768-779, 1985).

Thymosin was first isolated from the thymus by Abraham et al in 1965. Thymosin is a polypeptide composed of 28 amino acids having the ability to promote T-lymphocyte division and maturation and to strengthen cell immunity. This active polypeptide has already been widely used for the treatment of such illnesses as immunodeficiency, tumours, viral infections and autoimmune diseases.

IFN-α and THY therefore have similar and complementary bioactivities, such as immunity adjustment and the treatment of such diseases as viral infections and tumours. However, at present IFN-α and THY preparations are both produced singly, and IFN-α and THY are also administered singly in clinical application, which leads to high drug costs. In addition, the preparation of IFN-α and THY (and particularly their separation and purification) are relatively tedious.

There is therefore an urgent requirement in this field for the development of new drugs having the bioactivities of IFN-α and THY. Furthermore, there is also an urgent requirement in this field for the development of production processes involving low costs and/or simple steps.

One objective of this invention is to provide a new drug having the bioactivities of both IFN-α and THY, being a fusion protein of IFN-α and THY.

Another objective of this invention is to provide DNA which codes for the said fusion protein, a vector containing this DNA sequence, and a host cell containing this vector.

Another objective of this invention is to provide a method for producing the said IFN-THY fusion protein involving low costs and/or simple steps.

In the first aspect of this invention, a fusion protein is provided which contains: a human α interferon 2b amino acid sequence, a human thymosin amino acid sequence, and a 1-20 amino acid connecting peptide sequence between the human α interferon 2b amino acid sequence and the human thymosin amino acid sequence.

Preferably, the said connecting sequence contains 4-10 amino acids. Better still, the said fusion protein contains an amino acid sequence indicated by SEQ ID NO: 2.

In the second aspect of this invention, an isolated DNA molecule is provided, which codes for the above-mentioned fusion protein of this invention. Preferably, the said DNA molecule codes for a fusion protein containing the amino acid sequence indicated by SEQ ID NO: 2, and better still, the said DNA molecule includes the nucleotide sequence indicated by SEQ ID NO: 1.

In the third aspect of this invention, a vector is provided which contains the above-mentioned DNA molecule. Preferably, the said vector includes the recombinant Bobyx Mori Nuclear polyhedrosis virus BacIT CGMCC No. 0504. Also provided is a host cell containing the above-mentioned vector.

In the fourth aspect of this invention, a method is provided for producing the fusion protein of this invention, which includes the steps:

Culturing the above-mentioned host cells under suitable conditions for expressing the said fusion protein, or rearing animals containing the above-mentioned host cells, thus expressing the said fusion protein; and

Isolating the said fusion protein.

In the fifth aspect of this invention, a pharmaceutical composition is provided, which includes a pharmaceutically acceptable carrier or excipient or diluent, as well as an effective dose of the fusion protein of this invention.

Through broad and deep research, the present inventor fused the IFN gene and the THY gene together and carried out high efficacy expression in the lava of the silkworm, producing the IFN-THY fusion protein and thus retaining all the bioactivities of IFN while also adding the stimulating activity of THY in strengthening the immunity of the organism, resulting in a novel drug for the treatment of viral infections, inhibiting viral replication and strengthening the immunity of the organism.

Definitions

As used in this document, the technical terms "fusion protein of interferon and thymosin" and "IFN/THY fusion protein" etc. can be used interchangeably and both indicate a protein formed by the fusion of an interferon amino acid sequence and a thymosin amino acid sequence, in which it is possible to have or not to have a connecting peptide sequence between the two.

As used in this document, the technical term "interferon amino acid sequence" in the fusion protein refers to part of the amino acid sequence in the said fusion protein, this sequence being basically the same as the amino acid sequence in natural or modified interferon and having basically the same bioactivity as natural interferon. The interferon amino acid sequence preferably includes: the amino acid sequence of $\alpha$ interferon and even better $\alpha$ interferon 2b, and ideally natural human $\alpha$ interferon 2b.

As used in this document, the technical term "thymosin amino acid sequence" in the fusion protein refers to part of the amino acid sequence in the said fusion protein, this sequence being basically the same as the amino acid sequence in natural or modified thymosin and having basically the same bioactivity as natural thymosin. The thymosin amino acid sequence is preferably the natural human thymosin sequence.

The interferon and thymosin sequences can be sourced from human or non-human animals. However, they are preferably natural human sequences.

As used in this document, the technical term "connecting peptide" refers to a short peptide playing a connecting role between the interferon amino acid sequence and the thymosin amino acid sequence. The length of the connecting peptide is usually 1-20 amino acids, is preferably 3-10 amino acids and is ideally 4-6 amino acids. Technicians can design connecting peptides using conventional methods in this field. The connecting peptide will not usually affect or seriously affect the accurate folding and spatial conformation formed by the interferon amino acid sequence and the thymosin amino acid sequence.

A DNA sequence coding for the fusion protein of this invention can be completely synthesised artificially. Interferon and/or thymosin coding DNA sequences can also be obtained by the PCR amplification or synthesis methods, after which they are joined together forming a DNA sequence coded for the fusion protein of this invention.

After obtaining the DNA sequence coding for the fusion protein of this invention, it is inserted into a suitable expression vector, which in turn is inserted into a suitable host cell. Finally, the transformed host cell is cultured, and the novel fusion protein of this invention is obtained through separation and purification.

As used in this paper, the technical term "vector" includes plasmids, expression vectors, cloned vectors, viral vectors etc.

In this invention, all kinds of vectors already known in this field can be selected, such as commercially available vectors. When selecting a commercially available vector, the nucleotide sequence coding for the novel fusion protein of this invention can then be operationally connected to the expression control sequence, thus forming the protein expression vector.

As used in this paper, "can be operationally connected to" refers to a situation in which certain parts of a linear DNA sequence can influence the activity of other parts of the same linear DNA sequence. For example, if a signal peptide DNA acts as a precursor expression and participates in polypeptide secretion, the signal peptide (pre-secretion guide sequence) DNA can be operationally connected to polypeptide DNA; if the transcription of the control sequence is switched on, it can be operationally connected to the coding sequence; if a nucleotide binding location is moved to a position which enables it to be translated, it can be operationally connected to the coding sequence. Generally, "can be operationally connected to" signifies adjacency, while a pre-secretion guide sequence signifies adjacency in the reading frame.

In this invention, the technical term "host cell" includes prokaryocytes and eukaryocytes. Examples of commonly-used prokaryotic host cells include *Escherichia coli* and *Bacillus subtilis.* Commonly-used eukaryotic host cells include yeast cells, insect cells and mammalian cells. Preferably the host cell is a eukaryocyte and is better still a silkworm cell.

After obtaining the transformed host cell, this cell is cultured under conditions suitable for expressing the fusion protein of this invention, thus expressing the fusion protein. Thereafter, the expressed fusion protein is further separated.

However, an even better expression system is the silkworm expression system. It has now been shown that because of the protecting action of the large quantity of protein present in silkworm lymph and the actions of some unknown mechanisms, drugs produced by silkworm expression can directly enter the blood circulation when administered orally. Drugs produced by silkworm expression can therefore not only avoid pain and the danger of infection resulting from injection of the patient but can also avoid the processes of drugs separation and purification, greatly reducing the production costs of the drug.

In one practical example of this invention, the IFN-THY fusion gene was cloned into a baculoviral transfer plasmid to provide a recombinant insect baculovirus containing the IFN-THY fusion gene. By infecting silkworms with the said recombinant insect baculovirus, the silkworm can be made to produce the fusion protein of this invention.

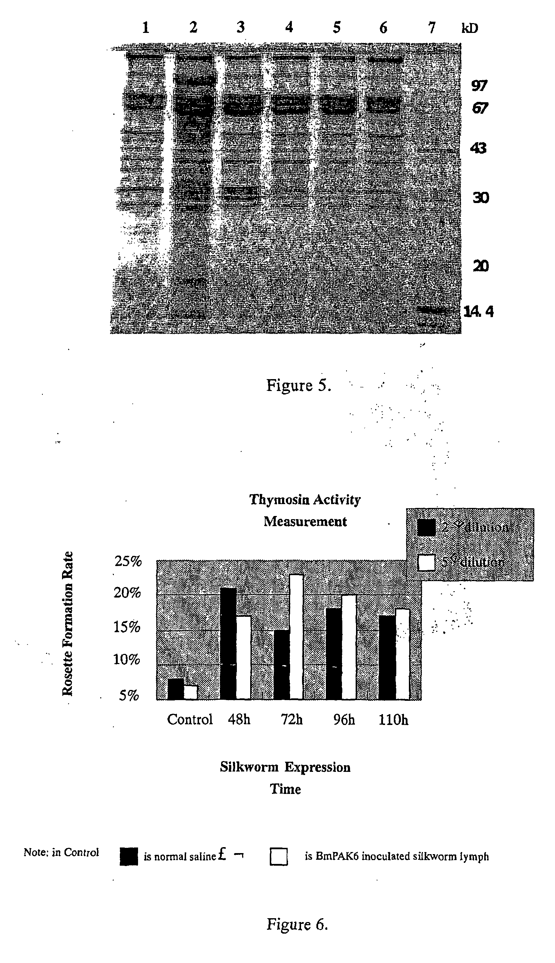

The IFN-THY fusion protein of this invention has the activity of IFN and the activity of THY. In one practical example, the supernatant liquid after centrifuging silkworm lymph at 5000 rpm for 5 minutes was taken and used directly for measurement of IFN activity. The results showed that the activity of the 96 hour expression sample IFN was $3.72 \times 10^4$ IU/ml, and the 110 hour expression sample was $3.10 \times 10^5$ IU/ml. The activity of the lymph supernatant liquid was also measured using the thymosin rosette method. The results showed that 24-110 hour expression sample rosette rates were in all cases above 10% (see Figure 6), showing that there was significant thymosin activity in all cases.

In another aspect of this invention, silkworms containing exogenous IFN-THY fusion genes are provided. Some or all the cells of the said silkworms contain exogenous exogenous [sic] IFN-THY fusion genes.

In another aspect of this invention, a pharmaceutical composition is also provided. The pharmaceutical composition of this invention includes an effective dose of the novel IFN-THY fusion protein of this invention, as well as at least one pharmaceutically acceptable carrier, a diluent or excipient. When preparing these compositions, the active ingredient is usually mixed with the excipient or is diluted with the excipient, or it is sealed in a gelatine capsule or a carrier present in the form of a pharmaceutical capsule. When the excipient plays a dilution role, it can be a solid, semi-solid or liquid material as an excipient, carrier or active ingredient medium. Pharmaceutical compositions can therefore be in the form of a tablet, pill, granule, solution, linctus or sterile injectable solution. Examples of suitable excipients include: lactose, glucose, sucrose, sorbose, mannitol, starch, microcrystalline cellulose, polyethylene pyrrolidone, cellulose, water etc. Preparations may also include: wetting agent, emulsifier, preservative (such as methyl and propyl hydroxybenzoate) and sweetener etc.

Compositions can be made up as single-element or multi-element preparations. All forms of preparation include a predetermined amount of the active material calculated to produce the hoped-for therapeutic effect as well as a suitable pharmaceutical excipient.

This pharmaceutical composition can be used according to the disease awaiting treatment and in a dose beneficial to the patient (depending on body weight and other relevant factors), which can be determined by medical personnel. Furthermore, the fusion protein of this invention can also be used in combination with other therapeutic drugs.

Summarising the above, the advantages of this invention are as follows:

(1) A fusion protein of IFN and THY prepared using a genetic engineering method avoids the disadvantages of the chemical cross-linking method, such as operational complexities, low rate of cross-linking, non-uniformity of the product, ready deactivation of the protein and relatively high costs;

(2) Simultaneously producing IFN and THY in a single vector avoids duplication of effort;

(3) Silkworm-expressed protein drugs can be taken directly by the oral route, avoiding the high cost operations of separating and purifying the protein, as well as eliminating the pain of injection and threat of infection for the patient;

(4) Silkworm larvae only require mulberry leaves for feed, the costs of which are very low.

IFN-THY fusion protein expressed by silkworm larvae has the bioactivity of IFN and also has the bioactivity of THY, making it a novel drug for the treatment of such diseases as viral infections and for strengthening the immunity of the organism.

Explanation of Figures:

Figure 1 is an electrophorogram of the IFN gene PCR amplification product.

Figure 2 shows the pET-IT expressed by *E. coli* containing the IFN-THY fusion gene.

Figure 3 shows the insect baculovirus transfer plasmid pBacPAK-IT.

Figure 4 shows the results of the second round phage-plaque-screened Southern blotting hybridisation.

Figure 5 is the SDS-PAGE analysis spectrum of the silkworm expression product, in which electrophoresis lanes 1-6 are samples at different times after infection by the recombinant virus, and electrophoresis lane 7 is the molecular weight standard substance.

Figure 6 is a bar chart of the THY activity of the silkworm expression product, in which the controls were physiological saline and silkworm lymph infected by BmPAK.

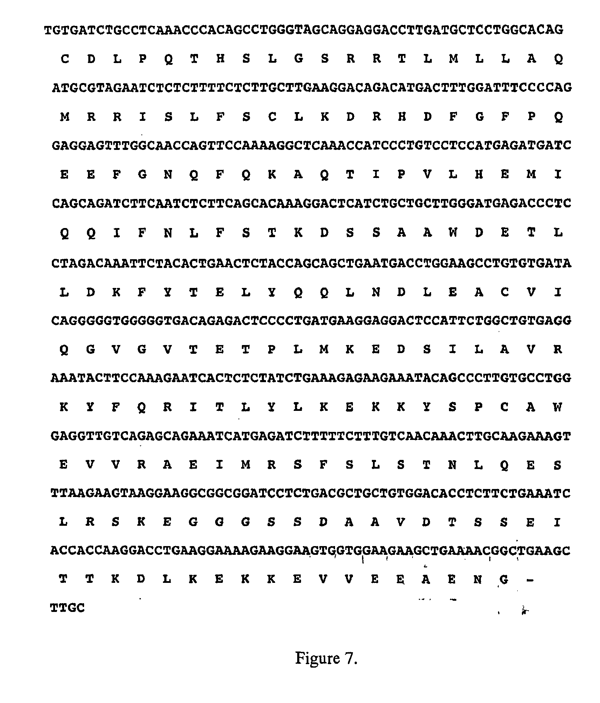

Figure 7 is the amino acid sequence of the IFN α 2b/THY fusion protein of this invention and the DNA sequence coding for it.

This invention is further elucidated below in combination with actual practical examples. It should be understood that these practical examples are only used to explain this invention and are not to be used to limit the scope of this invention. Where detailed conditions are not specified for the experimental methods in the practical examples listed below, they will usually be in accordance with conventional conditions, as for example the conditions quoted in Sambrook et al, Molecular Cloning: Laboratory Handbook (New York: Cold Spring Harbor Laboratory Press, 1989), or in accordance with the conditions recommended by the manufacturer.

**Practical Example 1**

**Synthesis of the IFN-THY fusion gene and construction of the *E-coli* expression plasmid**

In order for the IFN-THY fusion protein to have an accurate spatial structure, the IFN and THY were joined together with GGGGS as the connecting peptide. A pair of primers IFNPRI1 and IFNPRI2 were designed and synthesised according to the IFN genetic sequence and the connecting peptide nucleotide sequence, the nucleotide sequences of which were respectively:

```
IFNPRI1: 5' GCCATATGTG CGATCTGCCT CAAACC 3'(SEQ ID NO: 3)
```

```
IFNPRI2: 5' ATGGATCCAC CACCGCCTTC CTTACTTCTT AAACTTTC 3'(SEQ ID NO: 4)
```

Wherein IFNPRI1 is the N terminal coding sequence of the IFN mature protein; and IFNPRI2 is the complementary sequence of the IFN C terminal and connecting peptide code sequence.

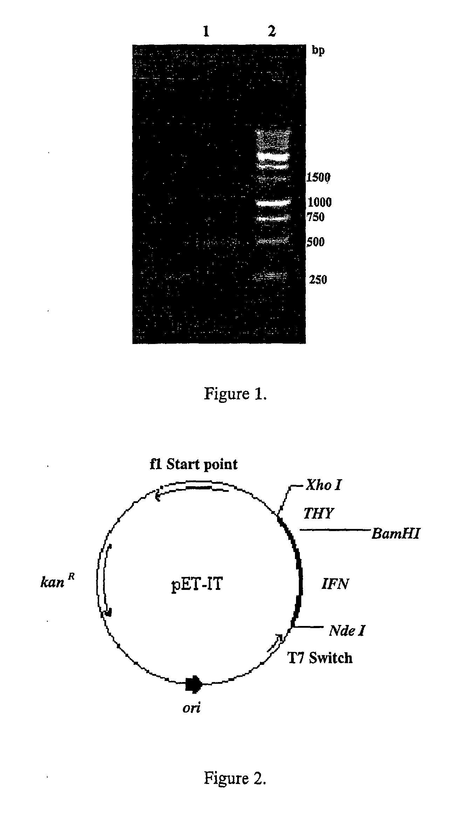

With IFNPRI1 and IFNPRI2 as primers, and the pSK-IFN plasmid containing the IFN gene as the template, PCR amplification was carried out on the IFN genetic sequence of the connecting peptide nucleotide sequence, introducing *Nde*I and *Bam*HI enzyme cleavage sites at the 5' terminal and 3' terminal respectively. The PCR conditions were: 94°C 1 min, 50°C 1 min, 72°C 1 min, total of 35 cycles, the first cycle denaturing at 94°C for 5 min, and the last cycle at 72°C extended to 10 min, with the result that approximately 500 bp of amplified product was obtained (Figure 1).

After the PCR product had undergone low melting point agarose purification, it was cloned to the pSK (+) *Eco*RV site, constructing the plasmid pSK-IFNL. Sequencing confirmed that the IFN gene and the connecting peptide nucleotide sequences were complete and accurate.

*Nde*I and *Bam*HI double enzyme cleavage was used to isolate the IFN and connecting peptide nucleotide sequences on the pSK-IFNL plasmid, and they were connected to pET-28a (+) which had previously undergone *Nde*I and *Bam*HI double enzyme cleavage, forming the plasmid pET-IFNL.

THY1 and THY2 fragments were designed and synthesised according to the THY amino acid sequence and in accordance with the eukaryotic affinity code, their nucleotide sequences being respectively:

```
THY1: 5' GGCGGATCCG GCTCTGACGC TGCTGTGGAC ACCTCTTCTG AAATCACCAC CAAGGACCTG
AAGGAAAAGA AGG 3'(SEQ ID NO: 5)
```

```
THY2: 5' CCAAGGACCT GAAGGAAAAG AAGGAAGTGG TGGAAGAAGC TGAAAACGGC TGAAGCTT
3'(SEQ ID NO: 6)
```

Wherein THY1 is the THY N terminal code sequence, introduced at the *Bam*HI enzyme cleavage site; and THY2 is the complementary sequence to the THY C terminal code sequence, introduced at the *Xoh*I enzyme cleavage site.

The THY1 and THY2 fragments were mixed at 1 : 1, dNTP and Klenow enzyme were added for mediation, and they were cloned to the pSK (+) *Eco*RV site, constructing the plasmid pSK-THY. Sequencing confirmed the synthesised THY genetic sequence to be accurate.

*Bam*HI and *Xoh*I double enzyme cleavage was used to isolate the THY gene on the pSK-THY plasmid, and it was connected to the pET-IFNL plasmid which had previously undergone *Bam*HI and *Xoh*I double enzyme cleavage, forming the *E. coli* expression plasmid pET-1T (Figure 2) containing the IFN-THY fusion gene. This plasmid contained the IFN gene, GGGGS connecting peptide code sequence and THY gene in sequence in the direction from 5' to 3'.

The IFN-THY fusion gene DNA sequence was as shown by SEQ ID NO: 1, wherein the code region was positions 1-594. The amino acid sequence of the coded IFN-THY fusion protein was as shown in SEQ ID NO: 2, with a total of 198 amino acids and a molecular weight of 22 kDa.

**Practical Example 2**

**Construction of the insect baculoviral transfer plasmid pBacPAK-IT containing the IFN-THY fusion gene**

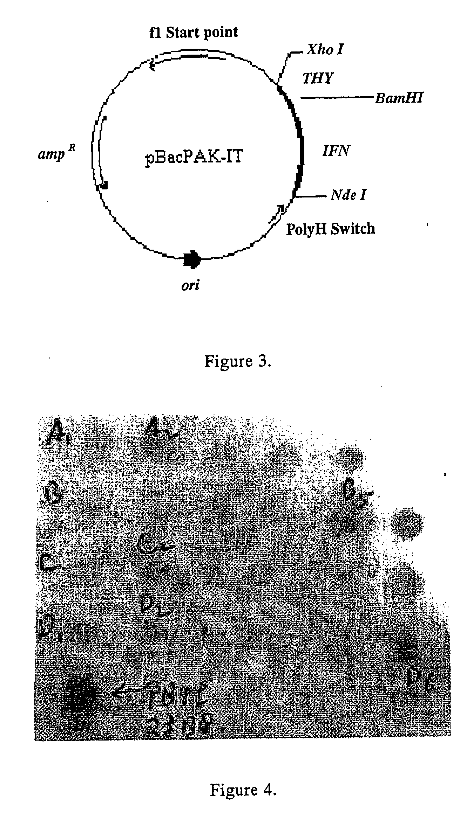

*Nde*I enzyme cleavage was first used on the *E-coli* expression plasmid pET-IT containing the IFN-THY fusion gene, and then mediation was carried out using the Klenow enzyme followed by *Xho*I enzyme cleavage. The IFN and THY fusion gene fragments were isolated using low melting point agarose, and were then connected to the pBacPAK8 which had previously undergone *Bam*HI enzyme cleavage and Klenow enzyme mediation followed by *Xho*I enzyme cleavage, forming the insect baculoviral transfer plasmid pBacPAK-IT (Figure 3) containing the IFN-THY fusion gene. This plasmid contained the IFN gene, GGGGS connecting peptide code sequence and THY gene in sequence in the direction from 5' to 3'.

**Practical Example 3**

**Obtaining recombinant insect baculovirus containing the IFN-THY fusion gene**

5 µl of insect baculoviral transfer plasmid pBacPAK-IT containing the IFN-THY fusion gene and 6 µl of modified viral BmBacPAK6 which had undergone *Bsu*36I enzyme cleavage and linearisation were taken, and 100 µl of serum-free TC-100 culture medium was added and mixed until uniform. 100 µl of serum-free TC-100 culture medium was added to 6 µl of Lipofectin and mixed until uniform. The two mixtures were then mixed together until uniform and allowed to stand for 15 minutes at room temperature. 800 µl of serum-free TC-100 culture medium was then added and mixed until uniform. Bm N cells which had previously been cultured in a 35 mm culture dish were washed twice with serum-free TC-100 culture medium, the transfer plasmid and Lipofectin mixture was added dropwise, and culturing was carried out for 4-5 days at 27°C. The supernatant fluid was taken and subjected to first round phage plaque screening. 5 µl of the supernatant fluid was taken and used to inoculate the Bm N cells in a 35 mm culture dish. After 1 hour the supernatant fluid was discarded and an equal volume of a mixture of TC-100 culture medium and low melting point agarose was added. After 4-5 days the plaques were lifted, and the Bm N cells were inoculated for 3-4 days, retaining the supernatant fluid. The cells were lysed with NaOH and used for Southern blotting hybridisation. The positive cloned supernatant fluid was subjected to second round phage plaque screening (see Figure 4, the plaque indicated by the arrow head). By amplification of the Bm N cells inoculated with the positive cloned supernatant fluid, it was possible to obtain a large quantity of recombinant baculovirus containing the IFN-THY fusion gene.

This recombinant baculovirus was named recombinant silkworm nuclear polyhedrosis virus (Bobyx Mori Nuclear polyhedrosis virus) BacIT, and it was stored at the General Microorganism Centre of the China Microorganism Species Conservation Management Committee (CGMCC, Beijing, China) on 6 November 2000, storage number CGMCC No. 0504.

**Practical Example 4, expression of the IFN-THY fusion gene in 5 day-old silkworm lava**

The recombinant baculovirus obtained by amplification in Practical Example 3 and containing the IFN-THY fusion gene was taken and injected into 5 day-old silkworm larvae, each lava being injected with approximately 10 µl. The expression silkworm lymph was taken at 24, 48, 72, 96 and 110 hours, and after centrifuging for 5 min at 5000 rpm, the supernatant fluid was taken. It was diluted tenfold with PBS at pH 7.4, and an equal volume of $2 \times$ protein sampling buffer liquid was added. Then 20 µl was taken and subjected to SDS-PAGE analysis. The results showed that beginning at 24 hours there was IFN-THY fusion protein expression (see Figure 5), and the IFN-THY fusion protein molecular weight was approximately 22 kDa.

**Practical Example 5**

**Measurement of IFN activity of the IFN-THY fusion protein**

The IFN activity was measured using the L929 cell (murine fibroblast strain) pathological change suppression method. The silkworm lymph supernatant fluid awaiting measurement was diluted to various degrees in a 96-well tray (50 μl per well, set up for 3 replications). Then 50 μl of L929 cell suspension ($4 \times 10^4$ cells) was added to each well, establishing a cell and virus control. Culturing was carried out for 24 hours at 37°C, when the liquid in the wells was discarded, and 100 μl of vesicula stomatitis virus (VSV) suspension was added to each well (250 TCID50/ml) (TCID: tissue culture infectious dose). 100 μl of culture medium was added to the cell control wells and cultured for 36 hours. Culturing was stopped when almost all the L929 cells in the virus control group wells had suffered pathological changes. The liquid in the wells was discarded, and 200 μl of 0.5% crystal violet solution was added to each well (crystal violet 0.5 g, NaCl 0.85 g, dissolved in 50 ml of anhydrous ethanol, 3 ml of methanol and 47 ml of distilled water), and this was left for 15 min at 37°C. The remaining dye was washed off with tap water, and 200 μl of decolourising fluid (mixture of 50 ml of ethylene glycol monoformaldehyde and 50 ml of distilled water) was added to each well. Agitation was carried out on an agitator, and when the dye had completely left the cells, colour comparison was carried out with a spectrophotometer at a wavelength of 540 nm. The IFN titre was calculated using the following formulae:

$$\log(\textit{IFN} \text{ titre}) = \frac{> [A]\text{end point}[A]\text{-}[A]\text{end point}}{>[A]\text{end point}[A]\text{-}<[A]\text{end point}[A]} + \log_2(\text{sample dilution reciprocal})$$

$$[A]\text{end point} = \frac{[A]\text{cell control} +[A]\text{virus control}}{2}$$

The results showed that the IFN activity of the 96 hour expression sample was $3.72 \times 10^4$ IU/ml, and for the 110 hour expression sample was $3.10 \times 10^5$ IU/ml.

**Practical Example 6**

**Measurement of THY activity of the IFN-THY fusion protein**

The THY activity was measured by the rosette method. Thymosin can act on the T lymph system, adjusting and strengthening the immune function of the organism. It was made to form rosettes with sheep erythrocytes through thymosin activation of -E thymocytes from fresh pig thymus[1]. Thymocytes which had reacted with lymph containing IFN-THY fusion protein after silkworm lava expression were counted under the oil immersion microscope: the pale blue and relatively large cells in the centre of the visual field were taken as lymphocytes (ie. thymocytes), each time counting 100 thymocytes, giving a total of 200. The numbers of cells therein forming E-rosettes were counted (thymocytes binding with more than 4 sheep erythrocytes), and the mean value was taken, that is the numbers read for the sample or the control. The THY activity was calculated according to the following formula:

$$\text{Sample activity=} \frac{\text{Mean reading of sample measurement tube-Mean reading of control tube}}{100} \times 100\%$$

The measurement results are shown in Table 1:

Table 1.

| IFN Activity of IFN-THY Fusion Protein | | |
|---|---|---|
| | Mean reading for rosette formation (%) | Activity (%) |
| Hank's fluid | 8 | |
| Lymph control without IFN-THY fusion protein | 7 | |
| 48 hour expression sample 2 × dilution | 21 | 13 |
| 48 hour expression sample 5 × dilution | 17 | 9 |
| 72 hour expression sample 2 × dilution | 15 | 7 |

[1] Translator's note: the grammatical structure of this sentence is difficult to follow, and its meaning may not have been rendered faithfully.

Table 1. (continued)

| IFN Activity of IFN-THY Fusion Protein | | |
|---|---|---|
| | Mean reading for rosette formation (%) | Activity (%) |
| 72 hour expression sample 5 × dilution | 23 | 15 |
| 96 hour expression sample 2 × dilution | 18 | 10 |
| 96 hour expression sample 5 × dilution | 20 | 12 |
| 110 hour expression sample 2 × dilution | 17 | 9 |
| 110 hour expression sample 5 × dilution | 18 | 10 |

The above results show that the expression samples from 48-110 hours all had significant THY activity. See Figure 6 for the bar chart of their activity.

All documents mentioned in this invention are cited as references in this application, just as if they had been individually cited. It should be furthermore understood that after reading the above instructional content of this invention, technicians in this field may make all manner of changes or amendments to this invention, and these equivalent forms likewise fall within the scope defined by the Claims appended to this application.

## Sequence Table

(1) General information:

(ii) Title of invention: Novel Interferon-Thymosin Fusion Protein and its Preparation Method and Uses

(iii) Number of sequences: 6

(2) Information on SEQ ID NO: 1:

(i) Sequence characteristics:
(A) Length: 604 bp
(B) Category: nucleic acid
(C) Chain type: double chain
(D) Topology: linear

(ii) Molecule type: DNA
(xi) Sequence description: SEQ ID NO: 1

```
TGTGATCTGC CTCAAACCCA CAGCCTGGGT AGCAGGAGGA CCTTGATGCT CCTGGCACAG   60
ATGCGTAGAA TCTCTCTTTT CTCTTGCTTG AAGGACAGAC ATGACTTTGG ATTTCCCCAG  120
GAGGAGTTTG GCAACCAGTT CCAAAAGGCT CAAACCATCC CTGTCCTCCA TGAGATGATC  180
CAGCAGATCT TCAATCTCTT CAGCACAAAG GACTCATCTG CTGCTTGGGA TGAGACCCTC  240
CTAGACAAAT TCTACACTGA ACTCTACCAG CAGCTGAATG ACCTGGAAGC CTGTGTGATA  300
CAGGGGGTGG GGGTGACAGA GACTCCCCTG ATGAAGGAGG ACTCCATTCT GGCTGTGAGG  360
AAATACTTCC AAAGAATCAC TCTCTATCTG AAAGAGAAGA AATACAGCCC TTGTGCCTGG  420
GAGGTTGTCA GAGCAGAAAT CATGAGATCT TTTTCTTTGT CAACAAACTT GCAAGAAAGT  480
TTAAGAAGTA AGGAAGGCGG CGGATCCTCT GACGCTGCTG TGGACACCTC TTCTGAAATC  540
ACCACCAAGG ACCTGAAGGA AAAGAAGGAA GTGGTGGAAG AAGCTGAAAA CGGCTGAAGC  600
TTGC                                                               604
```

(2) Information on SEQ ID NO: 2:

(i) Sequence characteristics:

(A) Length: 198 amino acids

(B) Category: amino acid

(D) Topology: linear

(ii) Molecule type: polypeptide

(xi) Sequence description: SEQ ID NO: 2

```
CDLPQTHSLG  SRRTLMLLAQ  MRRISLFSCL  KDRHDFGFPQ  EEFGNQFQKA   50
QTIPVLHEMI  QQIFNLFSTK  DSSAAWDETL  LDKFYTELYQ  QLNDLEACVI  100
QGVGVTETPL  MKEDSILAVR  KYFQRITLYL  KEKKYSPCAW  EVVRAEIMRS  150
FSLSTNLQES  LRSKEGGGSS  DAAVDTSSEI  TTKDLKEKKE  VVEEAENG    198
```

(2) Information on SEQ ID NO: 3:

(i) Sequence characteristics:

(A) Length: 26 alkaline groups

(B) Category: nucleic acid

(C) Chain type: single chain

(D) Topology: linear

(ii) Molecule type: oligonucleotide

(xi) Sequence description: SEQ ID NO: 3

```
GCCATATGTG  CGATCTGCCT  CAAACC                                26
```

(2) Information on SEQ ID NO: 4:

(i) Sequence characteristics:

(A) Length: 38 alkaline groups

(B) Category: nucleic acid

(C) Chain type: single chain

(D) Topology: linear

(ii) Molecule type: oligonucleotide

(xi) Sequence description: SEQ ID NO: 4

ATGGATCCAC CACCGCCTTC CTTACTTCTT AAACTTTC    38

(2) Information on SEQ ID NO: 5:

    (i) Sequence characteristics:

    (A) Length: 73 alkaline groups

    (B) Category: nucleic acid

    (C) Chain type: single chain

    (D) Topology: linear

    (ii) Molecule type: oligonucleotide

    (xi) Sequence description: SEQ ID NO: 5

GGCGGATCCG GCTCTGACGC TGCTGTGGAC ACCTCTTCTG AAATCACCAC    50
CAAGGACCTG AAGGAAAAGA AGG    73

(2) Information on SEQ ID NO: 6:

    (i) Sequence characteristics:

    (A) Length: 58 alkaline groups

    (B) Category: nucleic acid

    (C) Chain type: single chain

    (D) Topology: linear

    (ii) Molecule type: oligonucleotide

    (xi) Sequence description: SEQ ID NO: 6

CCAAGGACCT GAAGGAAAAG AAGGAAGTGG TGGAAGAAGC TGAAAACGGC    50
TGAAGCTT    58

**Claims**

1.  A fusion protein, **characterised by** including: a human α interferon 2b amino acid sequence, a human thymosin amino acid sequence and a 1-20 amino acid connecting peptide sequence between the human α interferon 2b amino acid sequence and the human thymosin amino acid sequence.

2.  Fusion protein according to Claim 1, **characterised by** the said connecting sequence containing 4-10 amino acids.

3.  Fusion protein according to Claim 1, **characterised by** the said fusion protein containing the amino acid sequence indicated by SEQ ID NO: 2.

4.  An isolated DNA molecule, **characterised by** its coding for the fusion protein according to Claim 1.

5.  DNA molecule according to Claim 4, **characterised by** including the nucleotide sequence indicated by SEQ ID NO: 1.

6.  A vector **characterised by** including the DNA molecule according to Claim 4.

7.  Vector according to Claim 6 **characterised by** including the recombinant Bobyx Mori Nuclear polyhedrosis virus BacIT CGMCC No. 0504.

8.  A host cell **characterised by** including the vector according to Claim 6.

9.  Method for producing the fusion protein according to Claim 1, **characterised by** including the steps: culturing the host cells according to Claim 8 under suitable conditions for expressing the said fusion protein, or rearing animals containing the host cells according to Claim 8, thus expressing the said fusion protein; and isolating the said fusion protein.

10. A pharmaceutical composition **characterised by** including a pharmaceutically acceptable carrier or excipient or diluent, as well as an effective dose of the fusion protein according to Claim 1.

Figure 1.

Figure 2.

Figure 3.

Figure 4.

Figure 5.

Note: in Control ■ is normal saline £ ¬ □ is BmPAK6 inoculated silkworm lymph

Figure 6.

```
TGTGATCTGCCTCAAACCCACAGCCTGGGTAGCAGGAGGACCTTGATGCTCCTGGCACAG
  C   D   L   P   Q   T   H   S   L   G   S   R   R   T   L   M   L   L   A   Q
ATGCGTAGAATCTCTCTTTTCTCTTGCTTGAAGGACAGACATGACTTTGGATTTCCCCAG
  M   R   R   I   S   L   F   S   C   L   K   D   R   H   D   F   G   F   P   Q
GAGGAGTTTGGCAACCAGTTCCAAAAGGCTCAAACCATCCCTGTCCTCCATGAGATGATC
  E   E   F   G   N   Q   F   Q   K   A   Q   T   I   P   V   L   H   E   M   I
CAGCAGATCTTCAATCTCTTCAGCACAAAGGACTCATCTGCTGCTTGGGATGAGACCCTC
  Q   Q   I   F   N   L   F   S   T   K   D   S   S   A   A   W   D   E   T   L
CTAGACAAATTCTACACTGAACTCTACCAGCAGCTGAATGACCTGGAAGCCTGTGTGATA
  L   D   K   F   Y   T   E   L   Y   Q   Q   L   N   D   L   E   A   C   V   I
CAGGGGGTGGGGGTGACAGAGACTCCCCTGATGAAGGAGGACTCCATTCTGGCTGTGAGG
  Q   G   V   G   V   T   E   T   P   L   M   K   E   D   S   I   L   A   V   R
AAATACTTCCAAAGAATCACTCTCTATCTGAAAGAGAAGAAATACAGCCCTTGTGCCTGG
  K   Y   F   Q   R   I   T   L   Y   L   K   E   K   K   Y   S   P   C   A   W
GAGGTTGTCAGAGCAGAAATCATGAGATCTTTTTCTTTGTCAACAAACTTGCAAGAAAGT
  E   V   V   R   A   E   I   M   R   S   F   S   L   S   T   N   L   Q   E   S
TTAAGAAGTAAGGAAGGCGGCGGATCCTCTGACGCTGCTGTGGACACCTCTTCTGAAATC
  L   R   S   K   E   G   G   G   S   S   D   A   A   V   D   T   S   S   E   I
ACCACCAAGGACCTGAAGGAAAAGAAGGAAGTGGTGGAAGAAGCTGAAAACGGCTGAAGC
  T   T   K   D   L   K   E   K   K   E   V   V   E   E   A   E   N   G   -
TTGC
```

Figure 7.